# EUROPEAN PATENT APPLICATION

(11) **EP 2 218 771 A1**
(43) Date of publication of application: **18.08.2010**
(21) Application number: 08859975.8
(22) Date of filing: 05.12.2008
(51) Int. Cl.: C12M 1/00, C12N 15/09

(54) **NEEDLE**

(30) Priority: 10.12.2007 JP 2007318890
(71) Applicant: Olympus Corporation, Shibuya-ku Tokyo 151-0072 (JP)
(72) Inventor: TATEYAMA, Kiyohiko, Tokyo 192-8512 (JP); SASAKI, Yasuo, Tokyo 192-8512 (JP); IMAOKA, Yuka, Tokyo 192-8512 (JP)
(74) Representative: Winter, Brandl, Fürniss, Hübner, Röss, Kaiser, Polte - Partnerschaft
(86) International application number: PCT/JP2008/072195
(87) International publication number: WO 2009/075236

(57) **Abstract**

A needle (10) designed to be manipulated on a single cell includes a cantilever tip (54) including a support unit (60) and a tip unit (44) formed at a prescribed angle to the support unit, and a shaft (54) connected to the cantilever tip, the needle being attachable, via at least the shaft (56), to a tip drive apparatus (14) capable of moving the tip unit (44) in a prescribed direction.

## Description

### Technical Field

The present invention relates to a needle acting on a single cell.

### Background Art

WO 04/092369 discloses a microinjection method and apparatus that are designed to introduce a substance, such as genes, into cell. In the technique disclosed, the substance is electrically adsorbed to the distal end of a microneedle, which is a tip unit, and the microneedle is then inserted into a cell. A pulse voltage is applied to the microneedle, moving the substance from the distal end of the microneedle and introducing the substance into the cell. The microneedle is thrust into the cell as it is minutely moved by using a piezoelectric element that can expand and contract coaxially with the microneedle. This technique is to hold genes at the distal end of the microneedle, can introduce the genes into the cell in a low-invasive manner, increasing the survival rate of the cell.

However, the microneedle cannot penetrate the cell membrane in some cases. This is because the microneedle invades the cell at a very small volume and also because the cell membrane has fluidity. Even if the microneedle is moved to such a position where its distal end may penetrate the cell membrane, the cell membrane covers up the surface of the distal end, disabling the needle from piercing the cell membrane in some cases. Consequently, the tip cannot be stably driven and the substance cannot be introduced at a sufficient rate.

The technique disclosed in the above-identified document cannot observe a cell in a living state with high efficiency by supplying an electric current to the microneedle in order to give an electrical stimulus to the living cell.

### Disclosure of Invention

This invention has been made in view of the foregoing. An object of the invention is to provide a needle that has a tip unit that can introduce a substance into a cell in a low-invasive manner, thus maintaining a high cellular survival rate or can apply an electrical stimulus to the cell in order to observe the living cell with high efficiency.

According to an aspect of embodiments, there is provided a needle designed to be manipulated on a single cell, comprising:
a cantilever tip including a support unit and a tip unit formed at a prescribed angle to the support unit; and
a shaft connected to the cantilever tip,
**characterized in that** the needle is attachable, via at least the shaft, to a tip drive apparatus capable of moving the tip unit in a prescribed direction.

### Brief Description of Drawings

FIG. 1 is a diagram showing the overall configuration of a tip drive apparatus that holds a needle according to a first embodiment of this invention.
FIG. 2 is a diagram showing the characterizing section of the tip drive apparatus.
FIG. 3 is a diagram showing the configuration of the needle according to the first embodiment.
FIG. 4 is a diagram explaining the interference that may be caused, depending on the angle of the needle.
FIG. 5 is a diagram explaining the region in which the needle can move.
FIG. 6 is a block diagram showing the electrical configuration of the tip drive apparatus holding the needle according to the first embodiment.
FIG. 7 is a flowchart explaining a tip driving method using the needle according to the first embodiment.
FIG. 8 is a diagram presenting a microscope image of HelaS3 cells into which genes to express GFP fluorescent protein has been introduced by using the needle according to the first embodiment.
FIG. 9 is a diagram presenting an image of the HelaS3 cells, acquired through a phase contrast microscope, 24 hours after the genes had been introduced into the cell to express GFP fluorescent protein, by using the needle according to the first embodiment.
FIG. 10 is a diagram presenting a microscope image of the HelaS3 cells, acquired through a fluorescence observation, 24 hours after the genes had been introduced into the cell to express GFP fluorescent protein, by using the needle according to the first embodiment.

### Best Mode for Carrying Out the Invention

Some of the best modes for carrying out this invention will be described, with reference to the accompanying drawings.

### [First Embodiment]

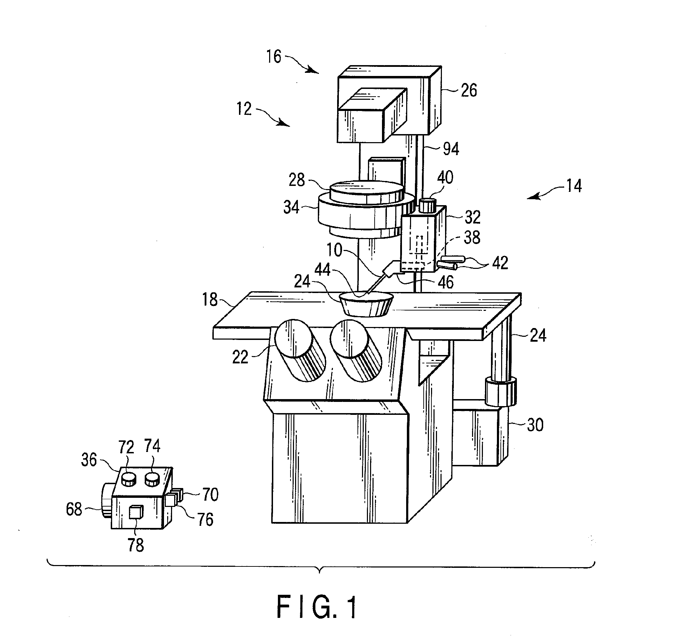

As shown in FIG. 1, a needle 10 according to a first embodiment of this invention is attached, via a tip drive apparatus 14, to an inverted microscope 12 through which to observe cells. So attached, the needle 10 is used.

The inverted microscope 12 has an illumination device 16, a microscope XY stage 18, a microscope XY stage handle 20, an objective lens (not shown), and an eyepiece 22. The illumination device 16 illuminates the cells set in on a dish 24. The microscope XY stage 18 moves the dish 24 in X direction and Y direction. When operated, the microscope XY stage handle 20 drives the microscope XY stage 18. The objective lens and the eyepiece 22 constitute an optical system through which to observe the light reflected from, or passing through, the cells mounted on the dish 24, or the fluorescent light emanating from the cells. At least the bottom of the dish 24 is made of transparent material such as glass, so that the cells may be observed.

The inverted microscope 12, which is a manually operable type, may be replaced by an electrically-driven type, in which the XY stage 18 is driven and controlled by a computer. Further, the inverted microscope 12 may instead be a type that has a CCD camera and can display images on a monitor.

The illumination device 16 has an illumination light source 26, a condenser lens 28, and an epi-illumination light source 30. The illumination light source 26 applies illumination light to the cells mounted on the dish 24, from the side opposite to the eyepiece 22. The condenser lens 28 receives the illumination light emitted from the illumination light source 26 and converges the light onto the cells. The epi-illumination light source 30 applies illumination light to the cells mounted on the dish 24, from the same side as the eyepiece 22.

The tip drive apparatus 14 according to this embodiment is composed of a main unit 32, a microscope adaptor 34, and an operation module 36. The microscope adaptor 34 is a unit that is attached to the condenser lens 28 of the main unit 32. As seen from FIG. 1, the main unit 32 is attached at the right side of the condenser lens 28, in front of the inverted microscope 12, where the eyepiece 22 is arranged. The operation module 36 is connected by a cable (not shown) to the main unit 32 and can be set at any desired position.

The main unit 32 has an adaptor holder unit 38, a Z-drive unit 40, and a needle-tip XY adjustment knob 42. The needle 10 having a tip unit 44, which should be driven, is attached to an adaptor 46. The adaptor 46 holding the needle 10 is attached to the adaptor holder unit 38. As the adaptor holder unit 38 is moved in the Z-direction, the Z-drive unit 40 drives the tip unit 44 in the Z-direction. The needle-tip XY adjustment knob 42 moves the adaptor holder unit 38 in X direction and Y direction, adjusting the XY position of the tip unit 44.

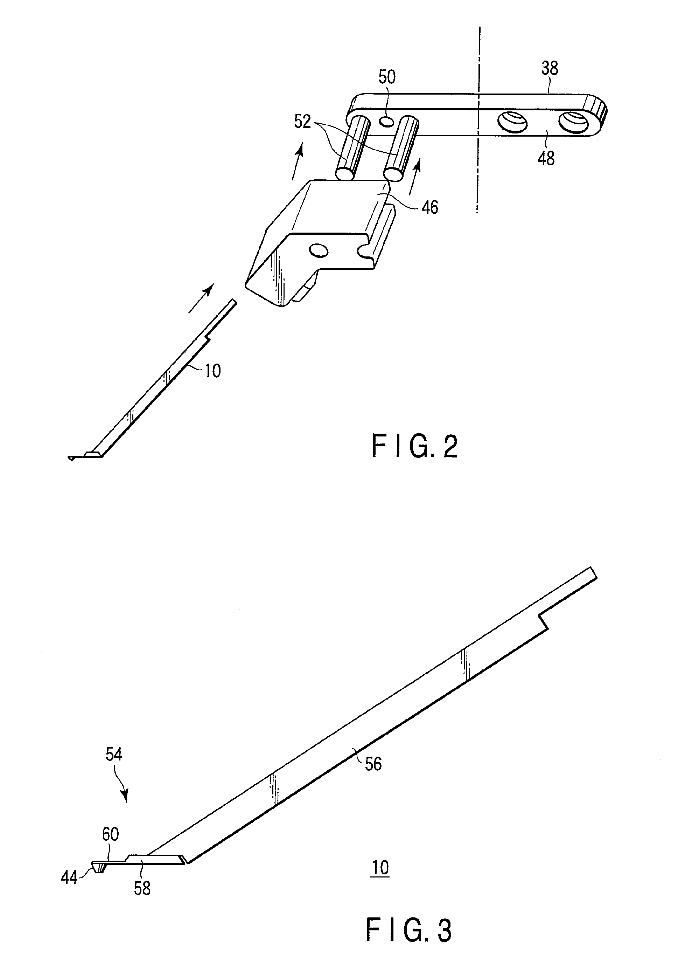

As shown in FIG. 2, the adaptor holder unit 38 has a Z-axis drive holder unit 48 configured to secure it to a linear movement mechanism (not shown) of the Z-drive unit 40 through a XY drive mechanism (not shown) (the needle-tip XY adjustment knob 42 drives the adaptor holder unit 38, in cooperation with the drive mechanism). Moreover, the adaptor holder unit 38 has an attachment member on the side opposite to the Z-axis drive holder unit 48 in the lengthwise direction. The attachment member is configured to attach the adaptor 46 to, and detached the same from, the adaptor holder unit 38. The attachment member is a magnet 50 if the adaptor 46 is made of metal or has a metal component. That section of the adaptor holder unit 38, which is illustrated on the right of the one-dot, dashed line shown in FIG. 2, is incorporated in the main unit 32. That is, the magnet 50 is provided outside the main unit 32. Near the magnet 50, fitting members 52 are provided, and can fit into the holes or grooves made in the adaptor 46 to set the adaptor 46 at a desired position. The fitting members 52 protrude toward the front of the inverted microscope 12. Therefore, the adaptor 46 is attached to the adaptor holder unit 38 by inserting into the holes or grooves from the front of the inverted microscope 12.

Another magnet 50 and other fitting members 52 may be provided on the back of the adaptor holder unit 38 so that the adaptor 46 may be attached to the adaptor holder unit 38 when the main unit 32 is secured to the left side of the condenser lens 28. Alternatively, the adaptor holder unit 38 may be replaced by another, depending upon the position at which the main unit 32 is secured.

As shown in FIG. 3, the needle 10 according to the embodiment, which is attached to the adaptor 46, is composed of a cantilever tip 54 and a shaft 56 holding the cantilever tip 54. The cantilever tip 54 has the tip unit 44 mentioned above. The cantilever tip 54 is connected to the distal end of the shaft 56.

The cantilever tip 54 has been manufactured by a silicon process and is composed of a silicon base unit 58, a flexible lever unit 60, and the above-mentioned tip unit 44. The silicon base unit 58 is a part to which another part, i.e., the shaft 56 is connected. The lever unit 60 extends from the silicon base unit 58 and has, for example, thickness of 2.7 µm, length of 240 µm and elastic constant of about 2 N/m. The tip unit 44 is formed at the free end of the lever unit 60, at an angle of about 90° to the lengthwise direction of the lever unit 60. The needle 10 according to this embodiment is first inserted into, and held in, the hole (not shown) made in the adaptor 46. Thereafter, the adaptor 46 holding the needle 10 is attached to the main unit 32. The needle 10, which is basically an expendable article and replaced frequently, can thus be replaced by a new one. Therefore, the tip drive apparatus 14 can be used over again, without the risk of contamination.

The cantilever tip 54 may be so configured to be attached directly to the main unit 32, while being held directly by hand, like most cantilever tips. In this case, the working efficiency is usually extremely low, because the cantilever tip 54 is very small as a whole as can be seen from the size of the lever unit 60. In the present embodiment, the cantilever tip 54 having the tip unit 44 is connected to the distal end of the shaft 56 configured to hold the cantilever tip 54. The shaft 56 is long enough (at least so long as it reaches the adaptor 46). Hence, if the shaft 56 is held by hand, the working efficiency is higher than in the case where the cantilever tip 54 is held by hand.

In this embodiment, the cantilever tip 54 and shaft 56, which constitute the needle 10, are separate members. They may be bonded together with a specific adhesive, forming an integral unit. Alternatively, they may be detachably coupled by means of a particular coupling/decoupling mechanism. If they are bonded, the needle 10 can be simple as a whole. If they are coupled, the same shaft 56 can be effectively utilized.

Further, in this embodiment, the shaft 56 is very thin (so thin that it would not hinder at least the function of the inverted microscope 12). Thus, the working efficiency can be increased, without impairing the function of the inverted microscope 12 (e.g., function of illuminating the sample).

The adaptor 46 is configured to hold the shaft 56 of the needle 10, at a prescribed angle and in a downwardly inclined position, when the adapter is attached to the main unit 32. Further, the cantilever tip 54 is adhered to the shaft 56, at a prescribed angle to the shaft 56. Moreover, as pointed out above, the tip unit 44 is provided, extending in a direction to intersecting with the lengthwise direction of the lever unit 60. The tip unit 44 is therefore held with its distal end directed downward, almost in the vertical direction, at the free end of the lever unit 60, as long as the adaptor 46 remains secured to the main unit 32.

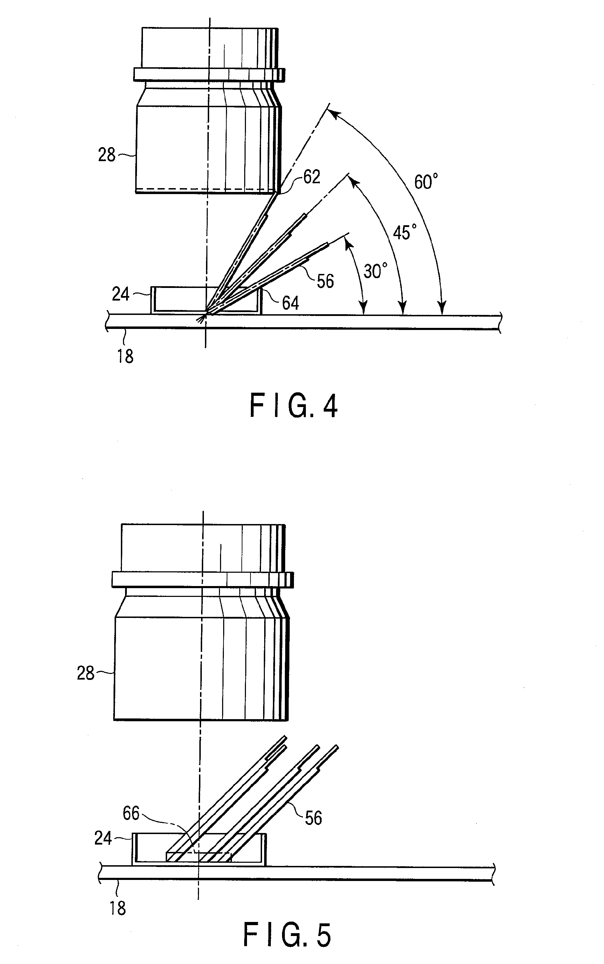

The angle at which the adaptor 46 holds the shaft 56 is determined as will be explained below. If the shaft angle indicated in FIG. 4 is too large, it will inevitably interfere with the condenser lens 28. Assume that the needle 10 is, for example, about 50 mm long. Then, the shaft 56 interferes with the condenser lens 28 if the shaft angle larger is larger than 60°. Conversely, if the shaft angle larger is too small, the shaft 56 inevitably interferes with the sidewall of the dish 24 as indicated by reference number 64 in FIG. 4. The dish 24 may be a glass-bottom of 35 mm dish that is usually used in cell culture. In this case, the shaft 56 interferes with the dish 24 if it is rotated upward by less than 30°. This is why the adaptor 46 holds the shaft 56 at the angle of 45° that is the intermediate value between 30° and 60°.

If the adaptor 46 holds the shaft 56 at the angle of 45°, there will be provided such a movement region 66 as indicated by a one-dot, dashed line shown in FIG. 5. The work can be proceeded, without the risk that the glass surface (having a diameter of about 14 mm) of the glass bottom dish of 35 mm interferes with the condenser lens 28 or the sidewall of the dish 24.

Thus, the angle at which the adaptor 46 holds the shaft 56 is determined to enable the needle 10 to have an sufficient movement region 46, in consideration of possible interference with the condenser lens 28 and the dish 24 used. The adaptor 46 has a hole (not shown) so shaped that the needles 10 may be inserted and held the shaft 56 at the angle so determined.

As shown in FIG. 1, the operation module 36 of the tip drive apparatus 14 has a Z-value adjustment handle 68, a speed setting dial 70, a minute-adjustment (up) button 72, a minute-adjustment (down) button 74, a movement setting dial 76, and a Z-value setting button 78.

The Z-value adjustment handle 68 and speed setting dial 70 are used to move coarsely the adaptor holder unit 38 in the Z-direction (in units of millimeters). As the Z-value adjustment handle 68 is rotated, the Z-drive unit 40 drives the adaptor holder unit 38 in the Z-direction, in accordance with the rotation of the Z-value adjustment handle 68. When operated, the speed setting dial 70 switches the distance by which to move the unit 38 as the Z-value adjustment handle 68 is rotated, from any one of the three values, i.e., long, intermediate and short, to another value.

The minute-adjustment buttons 72 and 74 and movement setting dial 76 are used to move minutely the adaptor holder unit 38 in the Z-direction (in units of microns). As the minute-adjustment (up) button 72 or minute-adjustment (down) button 74 is operated, the Z-drive unit 40 drives the adaptor holder unit 38 minutely in the Z-direction, in accordance with the operation of the button. The movement setting dial 76 switches the distance by which to move the unit 38 as the minute-adjustment button 72 or 74 is operated one time, from any one of the three values, i.e., long, intermediate and short, to another value.

The Z-value setting button 78 is a button, which may be pushed to instruct that any position in the Z-direction be stored. Even if the Z-value adjustment handle 68 or the minute-adjustment button 72 or 74 is operated, the adaptor holder unit 38 will never move down below the position stored by pushing the Z-value setting button 78 (toward the sample placed in the dish 24). The Z-value setting button 78 has a latch mechanism (not shown). Once depressed or turned on by the operator, the Z-value setting button 78 remains in the on state until it is depressed again. Hereinafter, the operation of the Z-value adjustment handle 68 and the minute-adjustment buttons 72 and 74 while the Z-value setting button 78 remains in the off state is called the "first mode," and the operation of the Z-value adjustment handle 68 and the minute-adjustment buttons 72 and 74 while the Z-value setting button 78 remains in the on state is called the "second mode."

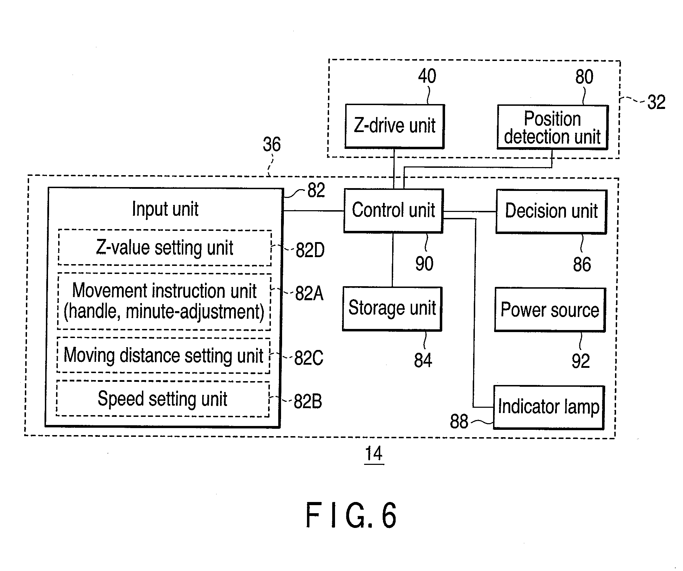

As shown in FIG. 6 illustrating the electrical configuration of the tip drive apparatus 14 according to this embodiment, the main unit 32 has, in addition to the Z-drive unit 40, a position detection unit 80 that is configured to detect the position of the adaptor holder unit 38. The position detection unit 80 may directly detect the position of the adaptor holder unit 38 by using optical means, or may indirectly detect the position of the adaptor holder unit 38 by detecting the distance by which the Z-drive unit 40 has been driven. Furthermore, the position detection unit 80 may be provided as a unit separated from the main unit 32.

The operation module 36 has an input unit 82, a storage unit 84, a decision unit 86, an indicator lamp 88, a control unit 90, and a power source 92.

The input unit 82 includes a movement instruction unit 82A, a speed setting unit 82B, a moving distance setting unit 82C, and a Z-value setting unit 82D. The movement instruction unit 82A outputs a movement instruction when the Z-value adjustment handle 68 is operated and the minute-adjustment button 72 or 74 is turned on. The speed setting unit 82B outputs a speed setting signal representing the moving speed set as the speed setting dial 70 is rotated. The moving distance setting unit 82C outputs a distance setting signal representing the distance set as the movement setting dial 76 is rotated. The Z-value setting unit 82D outputs a Z-value setting signal when the Z-value setting button 78 is turned on. The signals output from the input unit 82 are input to the control unit 90.

The storage unit 84 stores, as the Z-value, the position of the adaptor holder unit 38, which the position detection unit 80 detects when the Z-value setting button 78 is turned on. The decision unit 86 compares the position of the adaptor holder unit 38, which the position detection unit 80 has detected, with the Z-value stored in the storage unit 84, thereby determining whether the adaptor holder unit 38 has reached the position of the Z-value. The indicator lamp 88 blinks in response to the Z-value setting signal output from the Z-value setting unit 82D. Seeing the indicator lamp 88 blinking, the operator can confirm that the Z-value has been duly stored.

The control unit 90 controls the entire tip drive apparatus 14. The power source 92 supplies power to the components of the tip drive apparatus 14.

A tip driving method, which uses the tip drive apparatus 14 according to this embodiment, will be explained below.

Here, a case will be described, in which the tip drive apparatus 14 according to this embodiment is used to introduce a substance into cells being cultured in a culture solution filled in the dish 24.

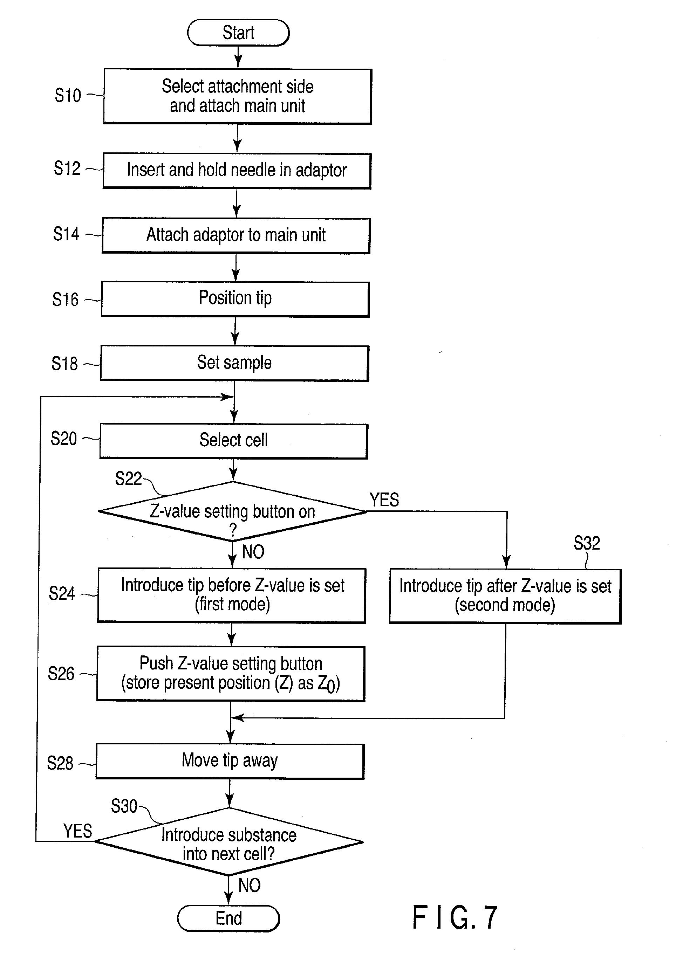

As shown in FIG. 7, the side to which the main unit 32 should be attached is selected, and the main unit 32 is then attached to the condenser lens 28 via the microscope adaptor 34 (Step S10).

Next, the needle 10 is inserted into, and held in, the adaptor 46 removed from the main unit 32 (Step S12). The adaptor 46 holding the needle 10 is attached to the adaptor holder unit 38 of the main unit 32, from the front of the inverted microscope 12 (Step S14).

Thereafter, the tip is positioned (Step S16). That is, while observing the needle 10, the operator brings the tip unit 44 formed at the distal end of the needle, to the center (i.e., view-field center) of the eyepiece (not shown). The operator accomplishes this by manipulating the needle-tip XY adjustment knob 42 of the main unit 32 and the Z-value adjustment handle 68 of the operation module 36. This manipulation is performed, not having the dish 24 mounted on the microscope XY stage 18. As for the Z-direction, the operator turns the speed setting dial 70 of the operation module 36, setting the long or intermediate distance, and then operates the Z-value adjustment handle 68, thereby lowering the tip unit 44 to a position where he or she can see the lever unit 60 of the cantilever tip 54.

When the tip unit 44 is so positioned, a sample is set, more precisely, the dish 24 is mounted on the microscope XY stage 18 (Step S18). This step is performed in the following sequence. First, the Z-value adjustment handle 68 of the operation module 36 is operated, moving the tip unit 44 at the distal end of the needle 10, to a safe region (upward in the Z-direction). An arm 94 (FIG. 1) of the inverted microscope 12 is then pulled back. As a result, the main unit 32 is moved as a whole. A space for sample setting is thereby provided. Then, the dish 24 (sample) is mounted on the microscope XY stage 18. Thereafter, the arm 94 of the inverted microscope 12 is moved to the initial position. Note that the dish 24 (sample) so set contains a substance in a dispersed state, which will be introduced into cells being cultured in a culture solution held in the dish 24.

Next, the cell into which the substance should be introduced is selected (Step S20). First, the operator manipulates the microscope XY stage handle 20, moving the microscope XY stage 18 and, thereby, arranging the cell held in the dish 24 into the view field of the microscope so that the cell may be observed. Thereafter, the operator actuates the Z-drive unit 40, moving the tip unit 44 of the needle 10 toward the cell from above. That is, while observing through the eyepiece 22, the operator lowers the tip unit 44 in the Z-direction until the lever unit 60 of the cantilever tip 54 comes into the view field and become visually confirmed. This is achieved by first turning the speed setting dial 70 of the operation module 36, setting the low speed, and then operating the Z-value adjustment handle 68. Since the cells in the dish 24 are not at the same height as the tip unit 44, the tip unit 44 is not focused and can hardly be observed. Therefore, the operator moves the tip unit 44 down in the Z-direction, using the lever unit 60 as index. This is because the lever unit 60 is larger than the tip unit 44 and therefore the lever unit 60 can be recognized generally, even if its image is not focused. After the lever unit 60 moves to a position where it is visually recognized, the operator adjust the position of the microscope XY stage 18 with respect to X direction and Y direction, while observing the cells through the eyepiece 22. The tip unit 44 is thereby set at a position that seems right above the cell into which to introduce the substance. Thus, the cell into which to introduce the substance is selected (determined).

The following operation depends on whether the Z-value has been set or not in the storage unit 84 of the operation module 36.

When the tip is driven for the first time, the Z-value has yet to be set in the storage unit 84 (Step S22). Therefore, the tip is introduced in the first mode (without using the Z-value) (Step S24). That is, the operator determines an optimal position in the Z-direction, while operating the Z-value adjustment handle 68 or the minute-adjustment button 72 or 74 and observing through the eyepiece 22, confirming "the distortion of the cell" or "the bending of the lever unit 60." At this point, the Z-value adjustment handle 68 is manipulated, while the speed setting dial 70 is turned, switching the speed from any one of the three values, i.e., high, medium and low, to another value. The minute-adjustment button 72 or button 74 is operated, while the movement setting dial 76 is turned, switching the distance from any one of the three values, i.e., long, intermediate and short, to another value.

As the tip unit 44 is thus moved down toward the bottom of the dish 24, lowering the distal end of the tip unit 44, the tip unit 44 contacts the cell in the dish 24. If the tip unit 44 is further lowered, the distal end of the tip unit 44 will pass through the cell membrane and penetrates the cell nucleus, forming a scar or hole in the membrane and nucleus. The substance dispersed in the dish 24 therefore flows into the cell through the formed scar or hole. The substance may flow into the cell, without forming a scar or hole, depending on the size of particles to introduce, if the channel coupled to a stretch receptor or the like is opened when the tip unit 44 deforms the cell, applying a physical stimulus to the cell. Thus, the substance is introduced.

When the substance is so introduced, the operator may push the Z-value setting button 78 of the operation module 36. Then, the control unit 90 of the operation module 36 determines that the Z-value setting button 78 has been pushed. In this case, the control unit 90 makes the storage unit 84 stores, as the Z-value representing an optimal position, the present position of the adaptor holder unit 38, which the position detection unit 80 has detected (Step S26). At this point, the control unit 90 turns on the indicator lamp 88.

Thereafter, the operator operates the Z-value adjustment handle 68 of the operation module 36, raising the needle 10 and, thus, moving up the tip unit 44 (Step S28). That is, the operator turns the speed setting dial 70 of the operation module 36, switching the speed to the medium speed or the low speed, and then operates the Z-value adjustment handle 68, raising the tip unit 44.

After the tip unit 44 is raised and pulled from the cell, and after a certain time has passed, the cell membrane is restored by itself and now contains the substance.

Assume that the tip unit 44 has been completely moved up (Step S28). Then, whether the substance should be introduced into the next sample cell is determined (Step S30). If NO, the operator turns off the power switch (not shown) of the main unit 32, terminating the tip driving method.

On the other hand, if the substance should be introduced into any other cell (Step S30), the method returns to Step S20. Thus, the substance may be introduced into other sample cells, one after another. That is, the operator manipulates the microscope XY stage handle 20, while making observation through the eyepiece 22, thereby actuating the microscope XY stage 18 and setting the tip unit 44 right above the cell into which to introduce the substance. In other words, the operator selects the cell into which the substance should be introduced (Step S20).

At the time the tip is driven for the second time, and so forth, the Z-value has already been set in the storage unit 84 (Step S22). Therefore, the tip is introduced in the second mode (by using the Z-value)
(Step S32). In this case, the Z-value has been set in the storage unit 84. Therefore, the operator can lower the tip unit 44 to an optimal position, merely by fully lowering the tip unit 44, without worrying about an excessive manipulation of the Z-value adjustment handle 68 and minute-adjustment buttons 72 and 74, once the tip unit 44 has been positioned in the horizontal direction. That is, the decision unit 86 of the operation module 36 compares the position of the adaptor holder unit 38, detected by the position detection unit 80, with the Z-value set in the storage unit 84, determining whether the adaptor holder unit 38 (tip unit 44) has reached the position of the Z-value. If the decision unit 86 determines that the adaptor holder unit 38 is found to have reached this position, the control unit 90 of the operation module 36 controls the Z-drive unit 40, preventing the same from moving down further, even if the Z-value adjustment handle 68 and the minute-adjustment buttons 72 and 74 are operated.

Since the optimal Z-position is set in the storage unit 84, the adaptor holder unit 38 (tip unit 44) may be automatically lowered to the optimal Z-position. That is, the handle operation in the second mode may be automated.

If the inverted microscope 12 is an electrically-driven type, a computer controls and drives the microscope XY stage 18. In this case, the inverted microscope 12 has a CCD camera or the like, and can display images on a monitor. If the inverted microscope 12 is such an electrically-driven type, not a manually operable type, any cell into which the substance should be introduced may be selected on the monitor screen, and the tip unit may be automatically moved to its position. In other words, the microscope XY stage 18 may be automatically adjusted in the XY direction.

Note that the substance to introduce into the cell may be anything that can be dispersed in the dish 24, such as genes, dyes, fluorescent reagent, e.g., quantum dots, ions, peptides, proteins or polysaccharides.

### [Examples]

A first example is HelaS3 cells which were immersed in a gene solution, and into which genes were introduced. The genes express GFP fluorescent protein. Whether the genes were successfully introduced or not can be confirmed by performing fluorescence observation.

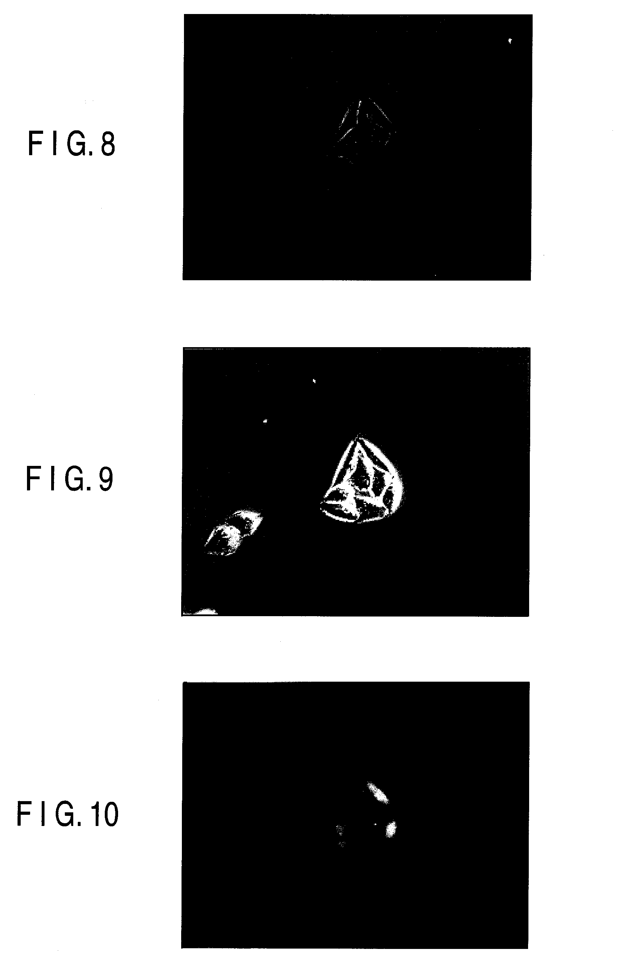

FIG. 8 presents a microscope image of the cells, obtained immediately after genes had been introduced. FIG. 9 and FIG. 10 are microscope images of the cells, acquired 24 hours after introducing the genes, which show whether the genes have been introduced into the cells. The image of FIG. 9 is one observed through a phase contrast microscope, showing the state the cells had 24 hours after the genes had been introduced.

FIG. 10 is one obtained through fluorescence observation. In the cell into which the genes had been successfully introduced, the genes well expressed, attaining intense fluorescent light. This proves that the genes were introduced into the cells at a very high efficiency.

As described above, the needle 10 according to this embodiment has the shaft 56 connected to the cantilever tip 54. This increases the efficiency of attaching the needle 10 to the tip drive apparatus 14. The substance can therefore be injected into the cell in such a low-invasive manner while maintaining such a high cellular survival rate as the conventional tip drive apparatus, and further with high reliability and high efficiency.

### [Second Embodiment]

In the first embodiment, only one needle 10 is attached, via the tip drive apparatus 14, to the inverted microscope 12. Nonetheless, a plurality of needles 10 may be attached at the same time via the tip drive apparatus 14. For example, needles 10 may be secured to both sides of the condenser lens 26.

If a plurality of needles 10 are used in this manner, tips can be used not only to introduce substances, but also to, for example, apply a potential difference between the tip units 44, thereby to give cells an electrical stimulus. The electrical stimulus is not necessarily be given exclusively by using a plurality of tip units 44. Rather, it can be given by applying a potential difference between a tip unit 44 and a particular electrode (e.g., glass bottom with ITO). If this is the case, the tip unit 44 had better be electrically conductive.

Thus, this embodiment can apply an electrical stimulus to cells, not only in such a low-invasive manner and maintaining the cell at a high survival rate, enabling the operator to observe the living cells with high efficiency.

This invention has been explained, with reference to various embodiments. The invention is not limited to the embodiments described above, nevertheless. Various changes and modifications can, of course, be made within the scope of this invention.

For example, the main unit 32 of the tip drive apparatuses 14 may be attached to a support unit supporting the condenser lens 28, not to the microscope adaptor 34 secured to the condenser lens 28 as in the embodiments described above.

## Claims

1. A needle designed to be manipulated on a single cell, **characterized by** comprising:
a cantilever tip (54) including a support unit (60) and a tip unit (44) formed at a prescribed angle to the support unit; and
a shaft (54) connected to the cantilever tip,
**characterized in that** the needle is attachable, via at least the shaft, to a tip drive apparatus (14) capable of moving the tip unit in a prescribed direction.

2. The needle according to claim 1, **characterized in that** the cantilever tip and the shaft are separate members.

3. The needle according to claim 2, **characterized in that** the cantilever tip and the shaft are bonded together with a specific adhesive.

4. The needle according to claim 2, **characterized in that** the cantilever tip and the shaft are coupled by a particular coupling/decoupling mechanism.

5. The needle according to claim 1, **characterized in that** the shaft functions as hand-held unit to attach the needle to the tip drive apparatus.

6. The needle according to claim 1, **characterized in that** the shaft is shaped not to impair the function of an inverted microscope (12) to which the tip drive apparatus is connected.
